# EUROPEAN PATENT SPECIFICATION

(11) **EP 3 980 102 B1**
(45) Date of publication and mention of the grant of the patent: **15.10.2025**
(21) Application number: 20731065.7
(22) Date of filing: 05.06.2020
(51) Int. Cl.: A61M 16/04, A61M 16/10

(54) **BREATHING PROTECTOR**
ATEMSCHUTZ
DISPOSITIF DE PROTECTION RESPIRATOIRE

(30) Priority: 07.06.2019 SE 1950676
(43) Date of publication of application: 13.04.2022
(73) Proprietor: Coloplast A/S, 3050 Humlebaek (DK)
(72) Inventor: BOLINDER, Nataniel, 21211 Malmö (SE); RAPPE, Johanna, 26868 Röstånga (SE); ROJAS, Maria, 21151 Malmö (SE)
(86) International application number: PCT/EP2020/065641
(87) International publication number: WO 2020/245367

(56) References cited:
- WO-A1-2010/060983
- WO-A2-2008/132222
- US-A- 4 582 058
- US-A1- 2014 150 779

## Description

### Field of the Invention

This invention pertains in general to the field of a breathing protector for use in a stoma of a laryngectomized or tracheotomised person, said breathing protector comprising a heat and moisture exchanger (HME) and said breathing protector having a plurality of inlets and at least one outlet, such that an airflow in use will pass from the surroundings of said person through said inlets, passing through said HME and to said outlet, into trachea of said person.

### Background of the Invention

A tracheostomy is a surgical procedure in which an opening is formed through the anterior surface of the neck into the trachea. The opening is referred to as a tracheostoma. A tracheostomy tube can be provided to extend between the tracheostoma and the trachea. A tracheostomy is performed, for example, when there is a malfunction, such as a result from injury or disorder, in respect of the nervous system or the respiratory passages, which malfunction results in an incapacity to obtain enough air. An inferior lung capacity or need of respiratory treatment may also result in a tracheostomy.

A laryngectomy is a surgical procedure, used for example to treat a carcinoma, which involves removal of the larynx or voice box and creation of a tracheostoma. A consequence of the procedure is that the trachea is no longer connected to the pharynx but is diverted to the tracheostoma. After this procedure, normal nasal function is not possible. In a subject whose breathing functions normally, the nose and the mucous membrane lining of the nasal cavity perform important functions in conditioning inhaled air. The convoluted passages and rich blood supply serve to increase both the temperature and humidity of the inhaled air to minimise the differential in these parameters with those of the surface of the lungs. Normally some heat and moisture is also captured from exhaled air prior to its release to the atmosphere. The mucous lining of the nasal passages also serves to remove particulate matter, such as fine dust particles, pollutants and microorganisms, from the inhaled air, and the action of cilia transports mucous and any particles away from the lungs.

When a patient has received a laryngectomy, in effect all inhaled air enters the lungs via the tracheostoma, and the nose is effectively not involved in the inhalation process. Exhaled air may pass through the tracheostoma or, if a voice prosthesis has been fitted, the stoma can be occluded so that the exhaled air is diverted through the voice prosthesis into the pharynx and the mouth, enabling the patient to speak. It is desirable that the flow of the exhaled air is controlled by means of a tracheostoma valve. In these situations, the valve can be arranged to remain open during breathing but can be closed to divert the airflow, through a small additional increase in exhaled airflow.

In this respect filter devices and breathing protectors have been developed to enable moisturizing of inhaled air and removal of small particles and bacteriological substances in said inhaled air. This is to resemble the functions of a nose. However, there are several complications related to the manufacturing of such devices. Firstly, the user of such devices is in need of good moisturizing and removal effect while keeping the size, such as the surface area, of the device as small as possible. Secondly, the moisturizing effect and removal effect is in need of large surface area, while not creating a too large resistance over the device. These criteria are contradictive, which the observant reader already has acknowledged.

US 5,487,382 describes an artificial nose with a housing and a hydrophilic filtering disc, further comprising a cap, which can be moved up and down to open/close windows in the housing. The artificial nose according to US 5,487,382 has to be actively moved from an open position to a closed position and vice versa, and has a very limited antibacteriological effect.

US 8,505,537 describes a breathing protector for use in a stoma of a laryngectomized or tracheotomised person. The breathing protector is provided with a closing member that may be activated to close the communication between said at least one inlet and said at least one outlet. The closing member is a member of, or attached to, a flexible cover. The resiliency of the flexible cover allows the flexible cover to be pressed down, inwardly, to closingly fit with a closing surface, such as a closing rib. A closing effect is obtained when the flexible cover is pressed against the closing rib. The breathing protector described in US 8,505,537 has many advantages, however there may exist occasions when there is a need of a lower breathing resistance and an improved humidification of the HME. An example of such occasion is during exercise.

US 8,991,394 describes a breathing protector comprising a filter housing, a first opening and a second opening. The first opening is located upstream of the second opening. An exchanging filter may be disposed in the filter housing and a valve seat may extend around the first opening. A valve member may be arranged to engage the valve seat in a closed position. However, even though this breathing protector may allow for a good moisturizing effect, as described above, sometimes there is a need of a lower breathing resistance and an improved humidification of the HME.

Hence, an improved breathing protector would be advantageous and in particular a breathing protector allowing for an excellent moisturizing effect in the space provided in the housing of the breathing protector, while still providing a small breathing protector. Furthermore, it would be advantageous to provide a breathing protector with a low resistance over said breathing protector such that the breathing protector may be comfortable to use e.g. during exercise. It would also be advantageous to provide a breathing protector that provides the possibility for a patient to close the breathing protector, such as during speech, with a low force and a breathing protector that is easily moved from an open to a closed state.

### Summary of the Invention

The invention is defined by the appended claims.

Accordingly, the present invention seeks to mitigate, alleviate or eliminate one or more of the above-identified deficiencies and to provide an improved breathing protector of the kind referred to. For this purpose the breathing protector has a housing with at least one inlet and at least one outlet, and wherein a heat and moisture exchanger (HME) is enclosed by said housing. The breathing protector is provided with a closing member arranged in a traversal plane to a central axis of the housing and distally of said HME. The closing member is adapted to closingly engage with the housing in a closed position and the closing member is provided with at least one inlet.

Advantageous features of the invention are defined in the dependent claims.

### Brief Description of the Drawings

These and other aspects, features and advantages of which the disclosure is capable of will be apparent and elucidated from the following description of embodiments of the present disclosure, reference being made to the accompanying drawings, in which
Fig. 1 is a perspective view of a breathing protector according to an embodiment of the present disclosure,
Fig. 2 is a cross sectional view of a breathing protector according to an embodiment of the present disclosure,
Fig. 3 is perspective view of a breathing protector according to an embodiment of the present disclosure,
Fig 4 is a perspective view of a breathing protector according to an embodiment of the present disclosure, and
Fig 5 is a perspective view of a breathing protector according to an embodiment of the present disclosure.

### Description of embodiments

The following description focuses on an embodiment of the present disclosure applicable to a breathing protector and in particular to a breathing protector for use in a stoma of a laryngectomized or tracheotomised person, where said stoma is communicating with trachea of said person. However, the invention is not limited to this application but may be applied to other technical fields in which one wishes to moisturizing an air stream and providing the possibility to close said air stream.

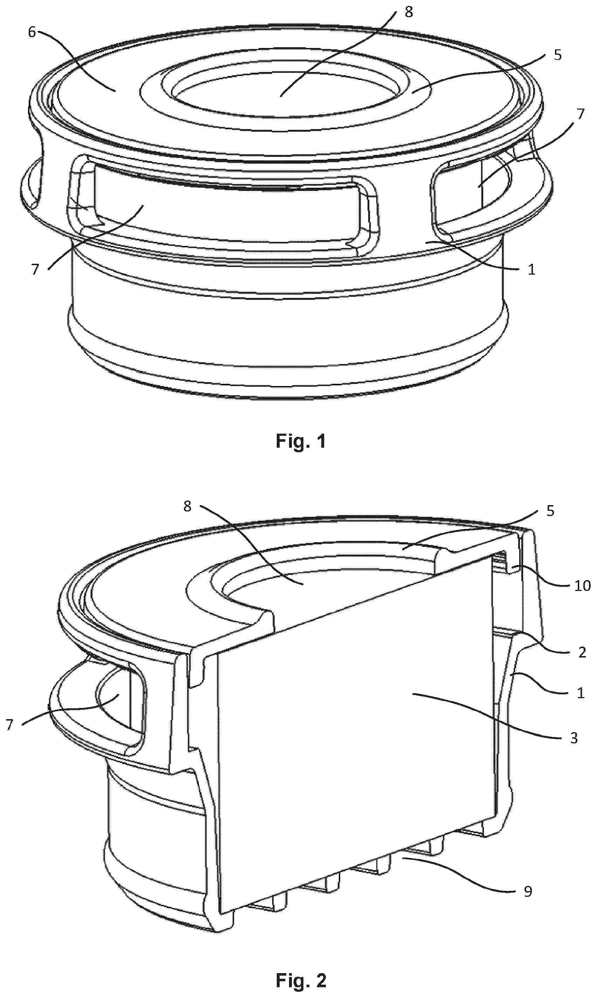

In an embodiment of the disclosure, which is illustrated in Figs. 1 and 2, a breathing protector is provided. The breathing protector is for use in a stoma of a laryngectomized or tracheotomised person. Said breathing protector comprises a housing 1 having at least one inlet 7 and at least one outlet 9. The at least one inlet 7 is located at an upper part of said housing 1, i.e. distal to the tracheostoma, and the at least one outlet 9 is located at a lower part of said housing 1, i.e. proximal to the tracheostoma. Said breathing protector further comprises a heat and moisture exchanger (HME) 3. The HME is arranged within, and enclosed by, said housing 1. As said housing 1 encloses the HME 3, a minimized contamination of the HME 3 may be assured, since the neck of the user is prevented from coming in contact with the HME 3.

As illustrated in Fig. 1, the breathing protector further comprises a closing member 6. The closing member 6 is not an integral part of the housing 1, but is separated from the housing 1. Thus, the closing member 6 is a separate closing member 6. The closing member 6 is arranged in a traversal plane to a central axis of the housing 1. It is positioned at an opening of the housing 1, which opening is located at an distal end of said housing 1, i.e. at the end furthest away from the neck of said person. The closing member 6 is arranged distally of said HME 3. Accordingly, the HME 3 is located within the housing 1, beneath the closing member 6.

The closing member 6 is adapted to closingly engage with said housing 1 in a closed position, such that the communication between the surrounding air and the trachea can be cut off. As further illustrated in Figs. 1 and 2, the closing member 6 is provided with at least one inlet 8. The present disclosure thus provides a breathing protector where an airflow in use will pass from the surroundings through the stoma, wherein or over which the breathing protector is arranged, into trachea of said person. The airflow will pass via said plurality of inlets 7,8 of the housing 1 and the closing member 6, through the HME 3, into trachea of the person through the at least one outlet 9 of the housing 1.

By providing at least one inlet 8 in the closing member 6, a larger airflow is achieved through the breathing protector. The humidification provided by said HME 3 is increased as air may flow into the breathing protector also through said at least one inlet 8. As air may flow through a larger number of inlets, the breathing protector is provided with a larger inlet surface area and the HME 3 is better utilized for humidification as the airflow also passes the part of the HME 3 that is provided underneath the closing member 6. Furthermore, due to the added airflow path through the closing member 6, the resistance to airflow through the breathing protector decreases, improving the overall functionality of the breathing protector. A higher resistance to airflow through the breathing protector is normally not a problem, but during exercise, a higher resistance may become a burden. The proposed breathing protector is thus made more tolerable for the user during heavy breathing. Thus, the disclosed breathing protector may be advantageous to use during exercise or at occasions when a heavier breathing is expected.

The at least one inlet 8 is preferably positioned centrally of said closing member 6. By providing the at least one inlet 8 centrally, it is easier to close the communication between the inlets 7, 8 and the at least one outlet 9 as it may be easier to activate the closing member 6 while at the same time blocking the at least one inlet 8 provided in the closing member 6. Thus, the airflow between the surrounding air and the trachea of the person is more intuitive and easily stopped. Furthermore, by providing the at least one inlet 8 centrally of said closing member 6, it may also facilitate for the user to apply a closing force at the right position of the closing member 6.

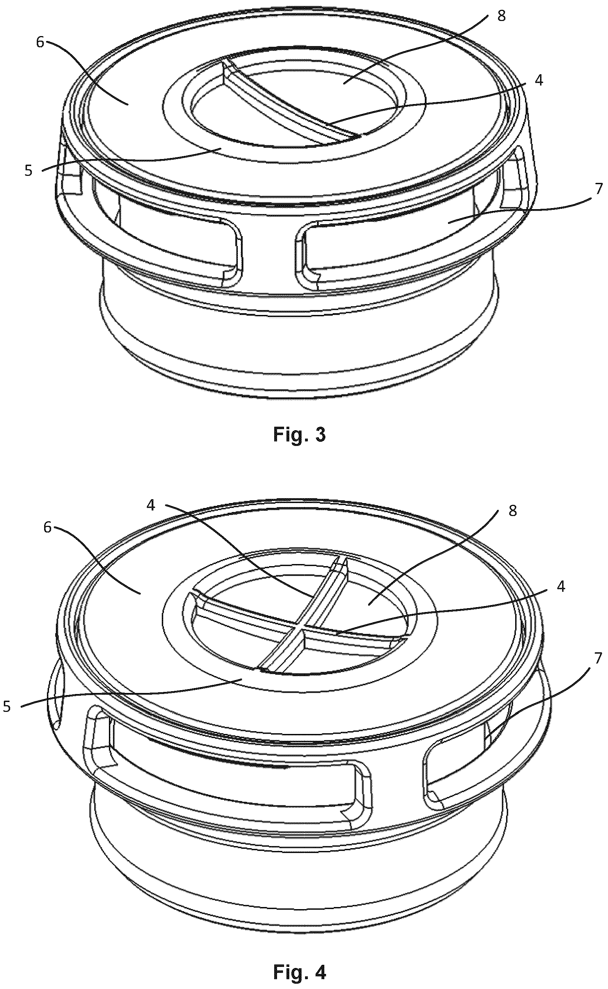

In one embodiment, as illustrated in Fig. 3, said at least one inlet 8 is provided with at least one dividing bar 4. In another embodiment, as illustrated in Fig. 4, said at least one inlet 8 is provided with a plurality of dividing bars 4. Fig. 4 illustrates an inlet 8 with two dividing bars 4 crossing each other, but it may be realized that the numbers of dividing bars 4 may be higher. By providing the inlet 8 with one or more dividing bars 4, the finger of a user is prevented from touching and contaminating the HME 3 when the user is pressing on the closing member 6 for closing the breathing protector. Thus, the HME 3 may be relieved from undue contamination from the finger. The one or more dividing bars 4 further prevent the HME 3 from protruding through the closing member 6, which might otherwise occur due to the pretension between the closing member 6 and the HME 3.

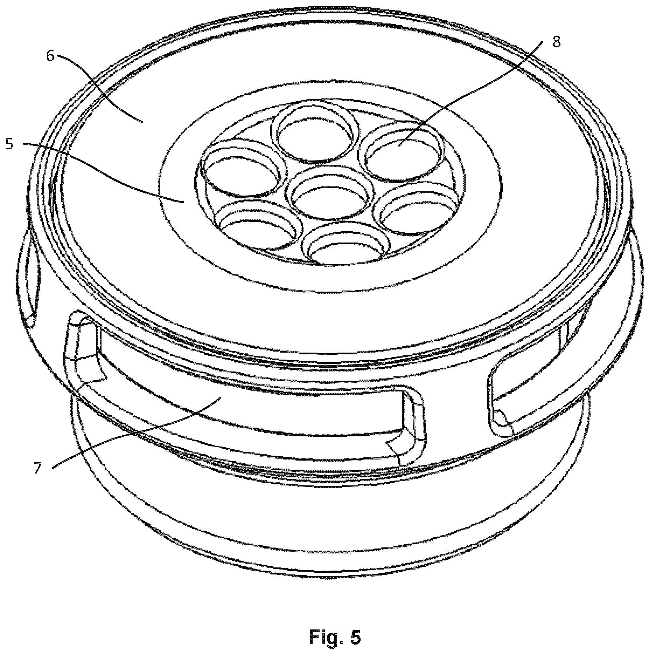

In another embodiment, the closing member 6 of the breathing protector may be provided with a plurality of inlets 8. This is illustrated in Fig. 5. The inlets 8 may be of any size and any numbers, they are only limited by the available area of the closing member 6, and by an area that is suitable to be covered by a finger. The size of the inlets 8 and the numbers of the inlets 8 may, in some embodiments, be chosen with regard to the manufacturing of the breathing protector. Accordingly, the closing member 6 according to the present disclosure may easily be adapted to prevailing manufacturing conditions. As is appreciated, each of these plurality of inlets 8 may also be provided with one or more dividing bars 4.

The closing member 6 may be fixed to the breathing protector in several ways. In one embodiment, the closing member 6 is glued to the HME in order to fix the closing member 6 to the breathing protector. In another, more preferred embodiment, the closing member 6 is attached to the breathing protector by protrusions extending from the closing valve 10 of the closing member 6. The protrusions extend radially out from the closing valve 10 and engage with the at least one inlet 7 provided in the housing 1. Thus, the protrusions extend into the at least one inlet 7 and thereby stop the axial movement in a distal direction of the closing member 6 and keep the closing member 6 in place.

As illustrated in each of Figs. 1 to 5, the closing member 6 is further provided with an opening interface structure 5. The opening interface structure 5 surrounds said at least one inlet 8 in the closing member 6 laterally. The opening interface structure 5 facilitates correct positioning of the finger during closure of the breathing protector. Accordingly, the breathing protector with an opening interface structure 5 on the closing member 6 is easier to close as the finger may feel where the at least one inlet 8 is located. Furthermore, the opening interface structure 5 may facilitate for the user to apply a closing force at the right location of the closing member 6. In one embodiment, the opening interface structure 5 is a guiding rim, extending upwards from the closing member 6. In another embodiment, the opening interface structure 5 is a recess in the closing member 6.

The closing member 6 is preferably made of a hard, non-flexible material preventing the closing member 6 from deforming and instead enabling the closing member 6 to react instantly to an applied force. The applied force enabling the closing member 6 to be activated to close the communication between said at least one inlet 7 and said at least one outlet 9. Accordingly, it may be possible to achieve a closing action of the breathing protector by applying a low force. At least an upper part of the housing 1 is preferably also made of a hard, non-flexible material preventing the upper part of the housing 1 from deforming. This may protect the internal structure of the breathing protector and may further prevent any unnecessary pressure to be applied to the HME 3.

As illustrated in Fig. 2, the housing 1 of said breathing protector is further provided with a closing valve seat 2. The valve seat 2 surrounds the HME 3. To complement the closing valve seat 2, the closing member 6 is provided with a closing valve 10. The closing valve 10 extends in a proximal direction, i.e. in a direction towards the user, from the closing member 6. The closing valve 10 is arranged laterally of the closing member 6 and thus extends in a direction down from the outer area of the underside of the closing member 6 into the housing 1. The structure of the closing member 6, with the closing valve 10, allows the closing valve 10 to be pressed down, inwardly, to closingly fit with the closing valve seat 2 of the housing 1, when the closing member 6 is pressed proximally, i.e. towards the user of the breathing protector. The closing valve 10 is thus adapted to seal against the closing valve seat 2. When the closing valve 10 seals against the closing valve seat 2, the communication between the at least one inlet 7 and the at least one outlet 9 is closed. Accordingly, a closing effect is obtained such that the at least one inlet 7 is blocked, or covered, by the closing valve 10 and not allowing air to pass through the at least one inlet 7 towards the at least one outlet 9.

The closing member 6 is activated by placing a finger over the at least one inlet 8 provided in the closing member 6 and by applying a force in a proximal direction, i.e. in a direction towards the user. The force in the proximal direction will then move the closing valve 10 in the same proximal direction, until it reaches the closing valve seat 2. At the same time, the finger that applies the force in the proximal direction is covering the at least one inlet 8 provided in the closing member 6, which will also close the communication between the at least one inlet 8 and the at least one outlet 9. Accordingly, it may be possible to achieve a closing action by activating the closing member 6 with a finger, which finger at the same time blocks, or covers, the at least one inlet 8 in the closing member 6. The closure of the breathing protector is activated by the user when the user intends to speak.

The previously disclosed embodiment provides a breathing protector with a compact design. A more compact design may be advantageous as a larger user population generally accepts these. Furthermore, the proposed embodiment provides the possibility for a patient to close the breathing protector, such as during speech, with a relatively low force. The structure of the disclosed breathing protector, with an externally accessible closing member 6, provides a distinct and well-defined closing. The structure of the breathing protector provides tactile confirmation of a closing state, i.e. an open or a closed state, of the breathing protector.

The material of the HME 3 should include flow passages therein, and should have an open structure in which the flow passages are randomly oriented. The material may comprise paper, foamed plastics, wadding made of different fibres, or combinations thereof. It may also be impregnated with a moisture absorbing substance. Furthermore, it is advantageous if the pores or interstices in the material do not have any special direction, such that the breathing air easily may pass through the material in a number of directions in order to achieve the intended deflection.

In one advantageous embodiment, the material of the HME 3 may be an HME foam. In such embodiments, the HME 3 may additionally functioning as a return spring for the closing member 6. The resiliency of the HME foam allows the closing member 6 to return to an open state of the breathing protector when pressure thereupon ceases. Thus, the user may press the closing member 6 to block, or cover, the at least one inlet 7 with said closing valve 10. Then the user may turn the breathing protector into a speaking mode/state, when the user wishes to speak, and simply release the pressure on the closing member 6 when the user wants to quit speaking and returning breathing protector into breathing mode/state.

In the embodiments described above, a breathing protector for use in a stoma of a laryngectomized or tracheotomised person has been described. This breathing protector comprises a housing 1, a closing member 6 and an HME 3. The housing 1 is provided with at least one inlet 7 and at least one outlet 9. The closing member 6 is arranged in a traversal plane to a central axis of the housing 1 and distally of said HME 3, and is adapted to closingly engage with said housing 1 in a closed position. Said closing member 6 is provided with at least one inlet 8, such that an airflow in use will pass from the surroundings of said person through said inlets 7, 8 to said at least one outlet 9, into trachea of said person. It is obvious to the skilled artisan, even if it has not been specifically disclosed, that the inlets and outlets may be divided into an increased amount by merely dividing the specific inlets and outlets already disclosed. The HME 3 is enclosed by said housing 1, such that said airflow will pass through said HME 3 when said airflow in use passes through said inlet 7, 8 to said outlet 9.

According to one aspect of the present disclosure, there is provided a method for humidifying air, during breathing through a stoma of a laryngectomized or tracheotomised person. The method comprises passing air from the surroundings into trachea of said person through a breathing protector. The breathing protector comprises a housing 1 provided with at least one inlet 7 and at least one outlet 9. The breathing protector further comprises an HME 3 and a closing member 6 provided with at least one inlet 8. The air will pass through the surroundings of said person through said plurality of inlets 7, 8 to said at least one outlet 9, into trachea of said person. The method further comprises moisturizing the air when the air is passed through the HME 3.

In another aspect, the present disclosure provides a method for closing a breathing protector for use in a stoma of a laryngectomized or tracheotomised person. The breathing protector has a housing 1 with at least one inlet 7 and at least one outlet 9. The breathing protector further comprises an HME 3. The breathing protector further comprises a closing member 6 provided with at least one inlet 8, such that an airflow in use will pass from the surroundings of said person through said inlets 7,8, pass through said HME 3 and to said at least one outlet 9, into trachea of said person. The housing 1 of the breathing protector is provided with a closing valve seat 2, which surrounds said HME 3. The closing member 6 is provided with a closing valve 10, which is adapted to seal against the closing valve seat 2. The method comprises closing said breathing protector by pressing said closing member 6 onto said housing 1, such that the closing valve 10 will cover, or block, the at least one inlet 7 provided in the housing 1. This is performed at the same time as the object, e.g. a finger, which is pressing said closing member 6 onto said housing 1, is covering, or blocking, the at least one inlet 8 provided in the closing member 6.

## Claims

1. A breathing protector for use in a stoma of a laryngectomized or tracheotomised person, said breathing protector comprising
a housing (1) having at least one inlet (7) and at least one outlet (9),
a heat and moisture exchanger, HME, (3), wherein said HME (3) is arranged within and enclosed by said housing (1), and
a closing member (6) arranged in a traversal plane to a central axis of the housing (1) at a distal open end of said housing (1) and arranged distally of said HME (3), wherein said closing member (6) is adapted to closingly engage with said housing (1) in a closed position and **characterized in that** said closing member (6) is provided with at least one inlet (8), such that an airflow in use will pass from the surroundings of said person through said inlets (7,8), pass through said HME (3) and further to said at least one outlet (9), into trachea of said person.

2. The breathing protector according to claim 1, wherein said at least one inlet (8) is positioned centrally of said closing member (6).

3. The breathing protector according to claim 1 or 2, wherein said at least one inlet (8) in the closing member (6) is provided with at least one dividing bar (4).

4. The breathing protector according to claim 3, wherein said at least one inlet (8) in the closing member (6) is provided with a plurality of dividing bars (4).

5. The breathing protector according to any of the previous claims, wherein said closing member (6) is provided with a plurality of inlets (8).

6. The breathing protector according to any of the previous claims, wherein said closing member (6) is provided with an opening interface structure (5) laterally surrounding said at least one inlet (8).

7. The breathing protector according to claim 6, wherein said opening interface structure (5) is a guiding rim.

8. The breathing protector according to claim 6, wherein said opening interface structure (5) is a recess.

9. The breathing protector according to any of the preceding claims,
wherein said closing member (6) is made of a non-flexible material.

10. The breathing protector according to any of the preceding claims,
wherein said housing (1) is provided with a closing valve seat (2) surrounding said HME (3), and said closing member (6) is provided with a closing valve (10), wherein said closing valve (10) is adapted to seal against the closing valve seat (2).

11. The breathing protector according to claim 10, wherein said closing member (6) during activation is arranged to close the communication between said at least one inlet (7) and the at least one outlet (9).

12. The breathing protector according to any of the previous claims, wherein the HME (3) comprises of an HME foam.

13. The breathing protector according to claim 10, wherein said HME foam is configured to act as a return spring for the closing member (6). (1)

## Patentansprüche

1. Atemschutz zur Verwendung in einem Stoma einer Person, bei der eine Laryngektomie oder Tracheotomie vorgenommen wurde, wobei der Atemschutz umfasst
ein Gehäuse (1) mit mindestens einem Einlass (7) und mindestens einem Auslass (9),
einen Wärme- und Feuchtigkeitsaustauscher, HME, (3), wobei der HME (3) in dem Gehäuse (1) angeordnet und davon umschlossen ist, und
ein Verschlusselement (6), das in einer Transversalebene zu einer Mittelachse des Gehäuses (1) in einem distalen offenen Ende des Gehäuses (1) und distal zu dem HME (3) angeordnet ist, wobei das Verschlusselement (6) dazu angeordnet ist, das Gehäuse (1) in einer geschlossenen Position verschließend in Eingriff zu nehmen und **dadurch gekennzeichnet, dass** das Verschlusselement (6) mit mindestens einem Einlass (8) bereitgestellt ist, so dass ein Luftstrom bei Verwendung aus der Umgebung der Person durch die Einlässe (7, 8), den HME (3) und ferner zu dem mindestens einen Auslass (9) und in die Luftröhre der Person strömen wird.

2. Atemschutz nach Anspruch 1, wobei mindestens ein Einlass (8) im Zentrum des Verschlusselements (6) positioniert ist.

3. Atemschutz nach Anspruch 1 oder 2, wobei der mindestens eine Einlass (8) in dem Verschlusselement (6) mit mindestens einer Trennleiste (4) bereitgestellt ist.

4. Atemschutz nach Anspruch 3, wobei der mindestens eine Einlass (8) in dem Verschlusselement (6) mit einer Vielzahl von Trennleisten (4) bereitgestellt ist.

5. Atemschutz nach einem der vorhergehenden Ansprüche, wobei das Verschlusselement (6) mit einer Vielzahl von Einlässen (8) bereitgestellt ist.

6. Atemschutz nach einem der vorhergehenden Ansprüche, wobei das Verschlusselement (6) mit einer Öffnungsschnittstellenstruktur (5) bereitgestellt ist, die den mindestens einen Einlass (8) lateral umgibt.

7. Atemschutz nach Anspruch 6, wobei die Öffnungsschnittstellenstruktur (5) ein Führungsrand ist.

8. Atemschutz nach Anspruch 6, wobei die Öffnungsschnittstellenstruktur (5) eine Aussparung ist.

9. Atemschutz nach einem der vorhergehenden Ansprüche, wobei das Verschlusselement (6) aus einem nicht flexiblen Material hergestellt ist.

10. Atemschutz nach einem der vorhergehenden Ansprüche, wobei das Gehäuse (1) mit einem Schließventilsitz (2) bereitgestellt ist, der den HME (3) umgibt, und das Verschlusselement (6) mit einem Schließventil (10) bereitgestellt ist, wobei das Schließventil (10) dazu angepasst ist, gegen den Schließventilsitz (2) abzudichten.

11. Atemschutz nach Anspruch 10, wobei das Verschlusselement (6) während der Aktivierung dazu angeordnet ist, die Verbindung zwischen dem mindestens einen Einlass (7) und dem mindestens einen Auslass (9) zu verschließen.

12. Atemschutz nach einem der vorhergehenden Ansprüche, wobei der HME (3) einen HME-Schaum umfasst.

13. Atemschutz nach Anspruch 10, wobei der HME-Schaum dazu ausgelegt ist, als eine Rückholfeder für das Verschlusselement (6) zu agieren.
(1)

## Revendications

1. Dispositif de protection respiratoire destiné à être utilisé dans une stomie d'une personne laryngectomisée ou trachéotomisée, ledit dispositif de protection respiratoire comprenant
un boîtier (1) présentant au moins une entrée (7) et au moins une sortie (9),
un échangeur de chaleur et d'humidité, HME, (3), dans lequel ledit HME (3) est disposé à l'intérieur dudit boîtier (1) et entouré par celui-ci, et
un élément de fermeture (6) disposé dans un plan transversal à un axe central du boîtier (1) au niveau d'une extrémité distale ouverte dudit boîtier (1) et disposé distalement par rapport audit HME (3), dans lequel ledit élément de fermeture (6) est conçu pour entrer étroitement en prise avec ledit boîtier (1) dans une position fermée et **caractérisé en ce que** ledit élément de fermeture (6) est pourvu d'au moins une entrée (8), de telle sorte qu'un flux d'air, en cours d'utilisation, passe de l'environnement de ladite personne à travers lesdites entrées (7, 8), passe à travers ledit HME (3) et en outre vers ladite au moins une sortie (9), dans la trachée de ladite personne.

2. Dispositif de protection respiratoire selon la revendication 1, dans lequel ladite au moins une entrée (8) est positionnée centralement par rapport audit élément de fermeture (6).

3. Dispositif de protection respiratoire selon la revendication 1 ou 2, dans lequel ladite au moins une entrée (8) dans l'élément de fermeture (6) est pourvue d'au moins une barre de séparation (4).

4. Dispositif de protection respiratoire selon la revendication 3, dans lequel ladite au moins une entrée (8) dans l'élément de fermeture (6) est pourvue d'une pluralité de barres de séparation (4).

5. Dispositif de protection respiratoire selon l'une quelconque des revendications précédentes, dans lequel ledit élément de fermeture (6) est pourvu d'une pluralité d'entrées (8).

6. Dispositif de protection respiratoire selon l'une quelconque des revendications précédentes, dans lequel ledit élément de fermeture (6) est pourvu d'une structure d'interface d'ouverture (5) entourant latéralement ladite au moins une entrée (8).

7. Dispositif de protection respiratoire selon la revendication 6, dans lequel ladite structure d'interface d'ouverture (5) est un rebord de guidage.

8. Dispositif de protection respiratoire selon la revendication 6, dans lequel ladite structure d'interface d'ouverture (5) est un évidement.

9. Dispositif de protection respiratoire selon l'une quelconque des revendications précédentes, dans lequel ledit élément de fermeture (6) est en matériau non flexible.

10. Dispositif de protection respiratoire selon l'une quelconque des revendications précédentes, dans lequel ledit boîtier (1) est pourvu d'un siège (2) de soupape de fermeture entourant ledit HME (3), et ledit élément de fermeture (6) est pourvu d'une soupape de fermeture (10), dans lequel ladite soupape de fermeture (10) est conçue pour assurer l'étanchéité contre le siège (2) de soupape de fermeture.

11. Dispositif de protection respiratoire selon la revendication 10, dans lequel ledit élément de fermeture (6) pendant l'activation est conçu pour fermer la communication entre ladite au moins une entrée (7) et ladite au moins une sortie (9).

12. Dispositif de protection respiratoire selon l'une quelconque des revendications précédentes, dans lequel le HME (3) comprend une mousse HME.

13. Dispositif de protection respiratoire selon la revendication 10, dans lequel ladite mousse HME est conçue pour faire office de ressort de rappel pour l'élément de fermeture (6).
(1)
